(19) Europäisches Patentamt

European Patent Office

Office européen des brevets

(11) **EP 1 369 082 A2**

(12) **EUROPEAN PATENT APPLICATION**

(43) Date of publication:
**10.12.2003 Bulletin 2003/50**

(51) Int Cl.[7]: **A61B 5/11**, A61B 5/02

(21) Application number: **03250579.4**

(22) Date of filing: **30.01.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **14.03.2002 JP 2002070440**

(71) Applicant: **SEIKO EPSON CORPORATION**
**Tokyo 160-0811 (JP)**

(72) Inventors:
• **Baba, Norimitsu, c/o Seiko Epson Corporation**
**Suwa-shi, Nagano-ken 392-8502 (JP)**
• **Yamaguchi, Kenji, c/o Seiko Epson Corporation**
**Suwa-shi, Nagano-ken 392-8502 (JP)**
• **Yamada, Sumio, c/o Seiko Epson Corporation**
**Suwa-shi, Nagano-ken 392-8502 (JP)**

(74) Representative: **Sturt, Clifford Mark et al**
**Miller Sturt Kenyon**
**9 John Street**
**London WC1N 2ES (GB)**

(54) **Physical activity measurement apparatus**

(57) To record physical activity and the amount of exercise, a CPU 130 calculates the user's heart rate while exercising from a pulse wave signal output from a pulse wave sensor unit 102 and an acceleration signal output from a acceleration sensor unit 140, calculates the number of steps taken by the user, and displays the results on an LCD 108. The CPU 130 continuously counts the number of steps based on the acceleration signal from the acceleration sensor unit 140, and displays the total number of steps taken by the user in one day on the LCD 108.

FIG._3

**Description**

The present invention relates to an apparatus for measuring the physical activity of the user.

It is known that the number of patients with heart disease in Japan has reached to approximately 800,000 in recent years. Heart disease is a dysfunction of the heart resulting from constriction or occlusion of the coronary artery leading to a drop in the blood supply to the heart or the heart stopping, and is generally classified according to the symptoms as either acute myocardial infarction or angina pectoris. Recent advances in medical technology have enabled treatment using such techniques as emergent intraveneous coronary thrombolisis that can reduce damage to the heart if applied within approximately three hours of the onset of the heart disease. If the symptoms are light the patient is often able to leave the hospital within approximately one week. The survival rate of patients with heart disease is thus rising.

Heart disease is believed to be closely related to so-called lifestyle-related diseases. If there is no improvement in the lifestyle-related disease the likelihood of the heart patient suffering a recurrence is extremely high and there will be no fundamental solution to the cause of the heart disease.

In order to improve the underlying lifestyle-related disease, heart patients are therefore commonly put on a regimen of rehabilitation including exercise within a few months (typically within six months) of the onset of the heart disease (referred to below as "heart disease rehabilitation"). If the symptoms were particularly serious, the rehabilitation always involves some degree of exercise, because the patient experiences a drop in physical strength without the rehabilitation. Even if the symptoms were light, the emphasis is on improving the lifestyle-related disease and exercise is included in the rehabilitation regimen in order to prevent a recurrence of heart disease. Treatment for high blood pressure, improvement for hyperlipemia, controlling diabetes, and not smoking are believed particularly effective for improving lifestyle-related disease, and it is therefore believed important for treatment to balance the patient's diet, exercise, and medicines.

Patients with heart disease have suffered damage to the heart to no small extent, and excessive exercise that overburdens the heart is extremely dangerous. This makes it important to monitor the load on the heart when the patient exercises, and this requires monitoring the patient's physical activity, including heart rate. Devices for measuring the heart rate as an indicator of physical activity when exercising have been developed so that patients can do the exercises required for heart disease rehabilitation at home.

A problem is that measuring the heart rate only while exercising does not enable the patient to accurately determine the total amount of physical activity performed during everyday life. More specifically, it is important to determine the total amount of activity from both normal activity during everyday life and exercising at the intensity level prescribed by the physician in order to improve the underlying lifestyle-related disease, and whether the prescribed amount and intensity of physical activity from exercising and daily life is appropriate is determined from the heart rate while exercising and the heart rate during normal physical activity. Continued use of such devices by users such as patients helps encourage and promote them the desire to exercise, and such devices are therefore also used to promote health and well-being.

The present invention is directed to these considerations, and an object of the invention is to provide a physical activity measurement apparatus for recording both the physical activity and the amount of activity of the user.

To achieve this object a physical activity measurement apparatus according to the present invention has a movement detection means for detecting user movement; a physiological reaction detection means for detecting the physiological reaction of the user; a physical activity data calculating means for calculating physical activity data for the user from detection results output by the physiological reaction detection means; an evaluation means for determining if the user started exercising; and storage means for storing detection results from the movement detection means and physical activity data from when the evaluation result becomes positive.

Thus comprised physical activity data for the user is stored to the storage means when the user exercises, and user movement data is stored to the storage means when the user is not exercising.

Preferably, the physical activity data calculating means calculates user physical activity data from detection results output by the movement detection means and physiological reaction detection means.

Further preferably, the storage means also stores detection results from the movement detection means from when the evaluation result becomes positive.

Yet further preferably, the physical activity measurement apparatus also has an input capturing means for capturing input from the user, and the evaluation means determines that the user started exercising based on a detection result from the movement detection means or when the input capturing means captures input from the user.

Yet further preferably, the storage means stores a predefined number of days of daily detection results from the movement detection means and one day of physical activity data from when the evaluation result becomes positive.

Yet further preferably, the storage means further stores one day of detection results from the movement detection means from when the evaluation result becomes positive.

Yet further preferably, the storage means stores daily detection results from the movement detection means, one day of physical activity data from when the evaluation result becomes positive, and one day of detection results from the movement detection means from when the evaluation result becomes positive, correlated to the date.

Further preferably the physical activity measurement apparatus also has a notification means for reporting detection results from the movement detection means until the evaluation result becomes positive, and reporting the physical activity data after the evaluation result becomes positive.

Preferably, after the evaluation result becomes positive the notification means further reports detection results from the movement detection means from when the evaluation result becomes positive.

The notification means can report to the user by displaying information on a display device, or by emitting information audibly.

Further preferably the movement detection means has an acceleration detection means for detecting acceleration based on user movement, and a number of steps calculating means for determining the number of user steps based on the detected acceleration. The physiological reaction detection means detects a pulse wave from the user, and the physical activity data calculating means calculates the heart rate as the physical activity data from the detected pulse wave.

Further preferably, the physical activity data calculating means calculates the heart rate from the detected pulse wave and acceleration.

Yet further preferably, the storage means further stores multiple heart rate ranges. The physical activity measurement apparatus also has a heart rate evaluation means for detecting which of the multiple heart rate ranges the heart rate calculated by the physical activity data calculating means is in, and a cumulative time calculation means for determining for each of the multiple heart rate ranges the cumulative time the calculated heart rate is within each range based on the evaluation result from the heart rate evaluation means. The storage means also stores the calculated cumulative times for each of the multiple heart rate ranges.

Yet further preferably, the storage means stores the total steps per day calculated by the number of steps calculating means and the cumulative time per day for each of the multiple heart rate ranges accumulated over a predefined number of days.

Further preferably, the storage means also stores the number of steps while exercising for one day from when the evaluation result becomes positive.

Yet further preferably, the physical activity measurement apparatus also has a display means for displaying a graphic according to the evaluation result of the heart rate evaluation means.

Yet further preferably, the physical activity measurement apparatus also has a heart rate range calculation means for determining a plurality of heart rate ranges according to a set heart rate that is set by the user.

Yet further preferably, the set heart rate is the upper limit of a prescribed heart rate range, and the heart rate range calculation means determines the plural heart rate ranges from the maximum heart rate and the pulse count width of the prescribed heart rate range.

Thus comprised the user can by looking at the displayed graphic determine the change in the heart rate while exercising and determine of the intensity of the activity is appropriate. From the cumulative times stored by the storage means the user can also determine while exercising, for example, for how long he has exercised at an appropriate intensity level.

Yet further preferably, the storage means further stores personal information including physical attributes of the user, and the physical activity measurement apparatus also has an amount of exercise calculation means for determining how much the user has exercised from detection results output by the movement detection means and the user-defined personal information. The storage means also stores a predefined number of days of the amount of exercise calculated for each day.

Yet further preferably, the storage means further stores personal information including physical attributes of the user. The physical activity measurement apparatus also has an amount of exercise calculation means for determining the amount of user exercise from the user-defined personal information and physical activity data from when the evaluation result of the evaluation means becomes positive. The storage means further stores a predefined number of days of the amount of exercise calculated for each day.

Thus comprised the user can quantify the amount of activity while exercising as an amount of exercise.

Yet further preferably, the storage means also stores a correlation table correlating basal metabolism, sex, and age information. The physical activity measurement apparatus also has a basal metabolism determination means for determining the user's basal metabolism from the correlation table and user-defined personal information, and a total energy consumption calculation means for calculating total energy consumption per day from the amount of exercise calculated by the amount of exercise calculation means and the basal metabolism determined by the basal metabolism determination means. The storage means also stores a predefined number of days of calculated total energy consumption data.

The user is thus able to know the total energy consumption per day including both energy consumed by exercise and the user's basal metabolism. As a result, the user can quantitatively determine the amount of physical activity per day from the total energy consumption. The predefined number of days is further preferably a unit of seven days.

Embodiments of the present invention will now be described by way of further example only and with reference to the accompanying drawings in which:-

Fig. 1 shows the appearance of a physical activity measurement apparatus according to the present invention and how it is used;

Fig. 2 shows the appearance of the physical activity measurement apparatus when the connector piece is disconnected;

Fig. 3 is a function block diagram of the physical activity measurement apparatus;

Fig. 4 is a side section view of the pulse wave sensor unit;

Fig. 5 describes the operation of the rectangular wave conversion circuit;

Fig. 6 is a flow chart of the interrupt process run by the CPU of the physical activity measurement apparatus;

Fig. 7 (a) is a graph of the pulse wave spectrum signal fmg, (b) is a graph of the acceleration spectrum signal fsg; and (c) shows the result of subtracting the acceleration spectrum signal fsg from the pulse wave spectrum signal fmg;

Figs. 8 to 11 show various changes in the display during the operation of the physical activity measurement apparatus;

Fig. 12 shows the segmentation of the heart rate into different ranges;

Fig. 13 shows the content recorded with the measurement results;

Fig. 14 shows the correlation between METS and walking speed;

Fig. 15 shows standard basal metabolism values according to sex and age;

Fig. 16 shows the back of the apparatus according to a sixth variation of the preferred embodiment; and

Fig. 17 shows a prompt displayed according to n eighth variation of the preferred embodiment of the invention.

Preferred embodiments of the present invention are described below with reference to the accompanying figures. The invention is described below as a device that can be worn on the wrist, and more specifically with application to a wristwatch.

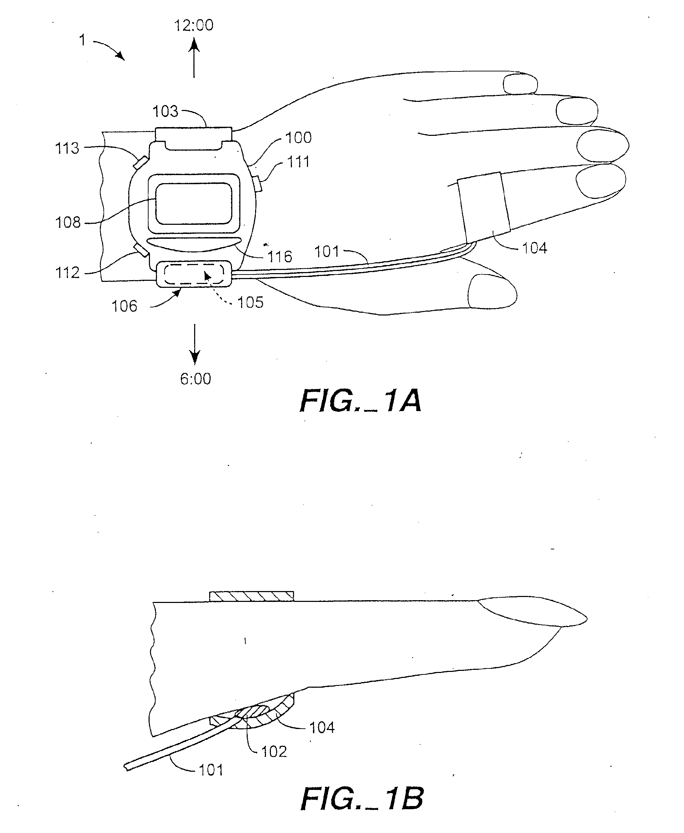

Fig. 1 shows the basic appearance of a wristwatch type physical activity measurement apparatus according to a preferred embodiment of the invention, and how the physical activity measurement apparatus is worn and used. This physical activity measurement apparatus 1 has a main unit 100 constructed like a wristwatch. The main unit 100 has a wristband 103 that wraps around the wrist of the user (such as a patient) and is fastened to the main unit 100 at the 12:00 and 6:00 positions of a typical wristwatch. The wristband 103 enables the main unit 100 to be worn on the user's wrist and removed as desired.

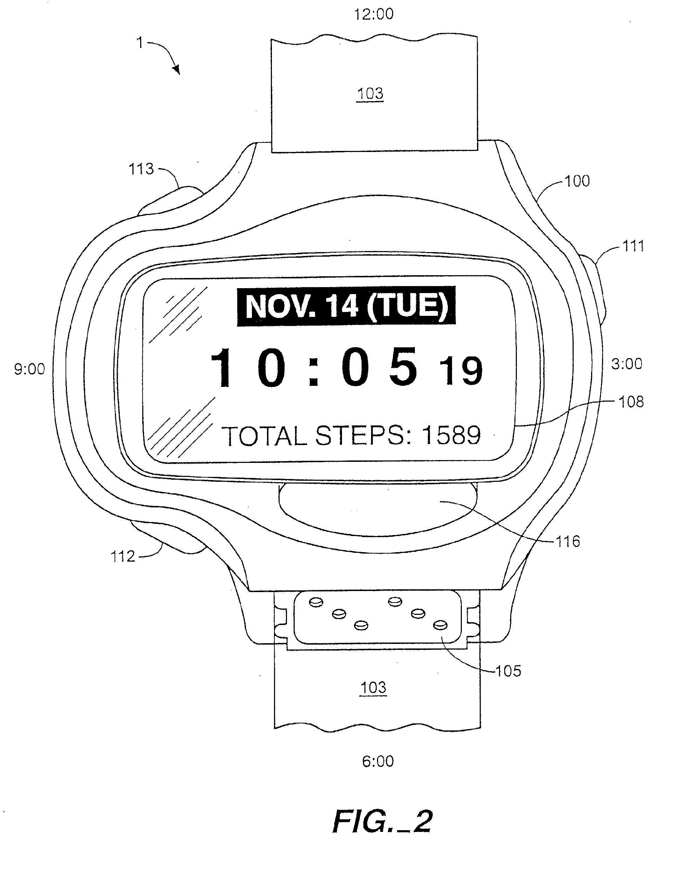

The main unit 100 has a liquid crystal display (LCD) 108 for displaying the date, current time, and number of steps taken by the user as shown in Fig. 2. A button switch 111 disposed at the 2:00 position on the outside of the main unit 100 can be pressed to change what is presented on the LCD 108 as described further below. Other button switches 112 and 113 disposed at the 7:00 and 11:00 positions, respectively, on the outside of the main unit 100 are used by the user to input information. Button switch 113 is also used to turn on the electroluminescent (EL) backlight of the LCD 108. A start/stop button switch 116 is also provided on the front of the main unit 100 (that is, the same side as the LCD 108).

The physical activity measurement apparatus 1 of this embodiment measures the heart rate as an indicator of physical activity, and measures the number of steps as an indicator of body movement. The start/stop button switch 116 is pressed by the user to start and stop these measurements.

A connector 105 is also disposed at the 6:00 position on the outside of the main unit 100 as shown in Fig. 1. A connector piece 106 can be connected to and disconnected from the connector 105. Connector piece 106 is connected to one end of cable 101 and a pulse wave sensor unit 102 is connected to the other end of the cable 101. The pulse wave sensor unit 102 is held at the base of one of the user's fingers by a sensor band 104. Because the connector piece 106 can thus be disconnected from the connector 105 with this configuration, the physical activity measurement apparatus 1 can be used as a wristwatch as shown in Fig. 2 by simply disconnecting the connector piece 106 from the connector 105.

A connector cover (not shown in the figure) is preferably placed over the connector 105 in order to protect the connector 105 when the cable 101 and pulse wave sensor unit 102 are disconnected from the connector 105. This connector cover is identical to the connector piece 106 except that the connector electrodes are not provided. The connector 105 is thus located at the front of the main unit 100 with this configuration for easy access by the user. It should also be noted that because the connector 105 does not protrude from the 3:00 position of the main unit 100 the back of the user's hand will not contact the connector 105, that is, the connector 105 will not restrict the movement of the user's hand.

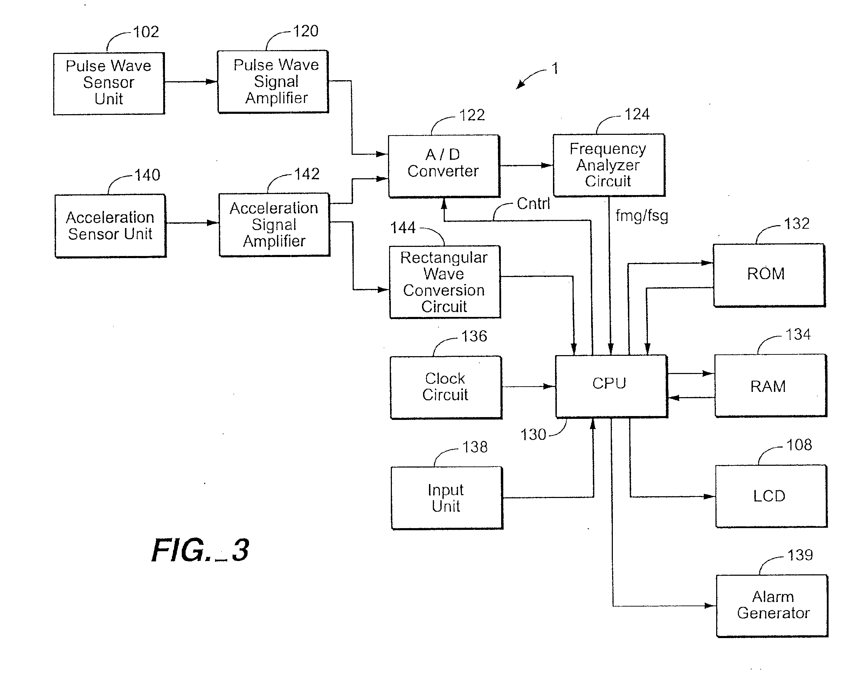

Fig. 3 is a function block diagram of this physical activity measurement apparatus 1. Referring to Fig. 3, the CPU 130 controls the operation of each unit of the physical activity measurement apparatus 1 and runs various operations. The ROM 132 is a rewritable ROM device such as EEPROM (Electrically Erasable Programmable ROM) for storing data and the control program run by the CPU 130. RAM 134 is used as working memory for the CPU 130 and temporarily stores data and the results of operations run by the CPU 130. Data stored in RAM 134 includes the date, number of steps, and personal information of the user. This personal information includes the sex and age of the user, as well as the height, weight, and other physical information.

The clock circuit 136 measures time and outputs the result to the CPU 130. The input unit 138 corresponds to the above described button switches 111 to 113 and 116, and outputs signals to the CPU 130 corresponding to user operations of the button switches. The LCD 108 displays information such as described above, and the specific information displayed is controlled by the CPU 130.

The alarm generator 139 generates an alarm at a volume controlled by the CPU 130. It should be noted that in addition to generating sound, the alarm generator 139 could have a vibrator for producing vibrations at an intensity controlled by the CPU 130.

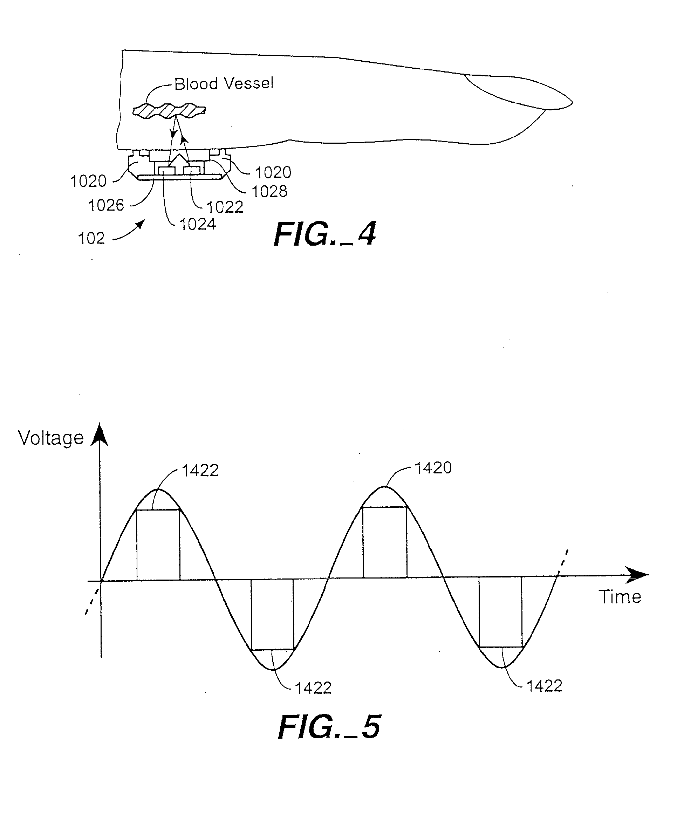

The pulse wave sensor unit 102 detects pulse waves which are organic activity of the user, and outputs a pulse wave signal to the pulse wave signal amplifier 120. More specifically, the casing of the pulse wave sensor unit 102 is sensor frame 1020 as shown in Fig. 4. An LED 1022, phototransistor 1024, and circuit board 1026 are disposed inside the sensor frame 1020. A glass or other type of light-transmitting plate 1028 is disposed at a direction of light emission by LED 1022 so that light from the LED 1022 passes through the transmission plate 1028. The circuit board 1026 is disposed opposite the transmission plate 1028. Light emitted from the LED 1022 thus is reflected from blood vessels below the skin of the user and detected by the phototransistor 1024. The reflected light is photoelectrically converted by the phototransistor 1024 to produce a pulse wave detection signal which is then output through cable 101 connected to the circuit board 1026 to the pulse wave signal amplifier 120 inside the main unit 100. Power is supplied to the pulse wave sensor unit 102 from a battery (not shown in the figure) inside the main unit 100 through cable 101.

The pulse wave signal amplifier 120 amplifies the pulse wave signal sent from the pulse wave sensor unit 102 and outputs to the A/D conversion circuit 122. The A/D conversion circuit 122 converts the received pulse wave signal from analog to digital signals only while a control signal is received from the CPU 130, and outputs to frequency analyzer circuit 124. More specifically, the CPU 130 outputs a control signal to the A/D conversion circuit 122 in order to operate the A/D conversion circuit 122, if this control signal is not output from the CPU 130 to the A/D conversion circuit 122, the pulse wave signal sent from the pulse wave signal amplifier 120 is discarded by the A/D conversion circuit 122.

The frequency analyzer circuit 124 buffers the pulse wave signal converted to a digital signal for a specific period, then applies a fast Fourier transform (FFT) to get the frequency component of the pulse wave signal, and outputs the result as pulse wave spectrum signal fmg to CPU 130.

The acceleration sensor unit 140 detects steps taken by the user as an indicator of user's body movement, and has an acceleration sensor for detecting the acceleration of repeated swinging of the users' arm as the user walks. The acceleration sensor is incorporated in the main unit 100, detects the acceleration of the arm swing in conjunction with the user walking, and outputs an acceleration signal to the acceleration signal amplifier 142. The acceleration signal amplifier 142 amplifies the received acceleration signal, and outputs to the A/D conversion circuit 122 and rectangular wave conversion circuit 144. As noted above the A/D conversion circuit 122 converts the received acceleration signal from analog to digital signals only while a control signal is applied from the CPU 130, and outputs to the frequency analyzer circuit 124.

More specifically, the A/D conversion circuit 122 alternately (i.e., time division) receives the pulse wave signal from the pulse wave signal amplifier 120 and the acceleration signal from the acceleration signal amplifier 142 for a specific period each, and outputs to the frequency analyzer circuit 124. The frequency analyzer circuit 124 receives the digitized acceleration signal for this specific period, applies an FFT operation to obtain the frequency component of the acceleration signal, and outputs the result as acceleration spectrum signal fsg to the CPU 130.

The pulse wave spectrum signal fmg and acceleration spectrum signal fsg output from the frequency analyzer circuit 124 are thus alternately input to the CPU 130. The CPU 130 calculates the pulse wave from the received pulse wave spectrum signal fmg and acceleration spectrum signal fsg, and obtains the heart rate, and runs a process to obtain the number of steps from the acceleration spectrum signal fsg. This process is described more fully below.

The rectangular wave conversion circuit 144 sequentially converts the shape of the acceleration signal sent from the acceleration signal amplifier 142 to produce a substantially rectangular wave. More specifically, the acceleration signal 1420 sent from the acceleration signal amplifier 142 is a substantially sine wave corresponding to the repeated swinging of the arm in conjunction with the user walking, and the rectangular wave conversion circuit 144 forms a rectangular pulse 1422 whenever the amplitude of the acceleration signal exceeds a specific threshold level. The rectangular wave conversion circuit 144 outputs a step detection signal to the CPU 130 each time a rectangular pulse 1422 is formed. The CPU 130 counts the number of step detection signals received to count the number of steps from the repeated swinging of the user's arm.

The threshold value used by the rectangular wave conversion circuit 144 to form these rectangular pulses can be desirably set. It should be noted, however, that because an acceleration signal is output from the acceleration sensor unit 140 even when the user moves his arm slightly with this configuration, the average amplitude of the acceleration signal output when the user's arm swings in conjunction with walking is preferably used for this threshold value. This removes the effects of slight movements of the arm, and thus more accurately detects the number of steps walked by the user.

The CPU 130 can determine the number of steps taken by the user using either of two ways: calculating the number of steps from the acceleration spectrum signal fsg output by frequency analyzer circuit 124, or counting the number of steps from the step detection signal output by rectangular wave conversion circuit 144.

In order to count the number of steps walked by the user each day while the heart rate is not being measured (that is, when not exercising), this embodiment of the invention counts the number of steps according to the step detection signal from the rectangular wave conversion circuit 144. When the heart rate is being measured, that is, while exercising, the number of steps is counted based on the acceleration spectrum signal fsg from frequency analyzer circuit 124 (referred to below as the "exercise steps"). When the user finishes exercising, the CPU 130 adds the exercise steps with the number of steps counted while not measuring the heart rate (that is, when not exercising). The combined number of steps is the number of steps per day ("total steps" below).

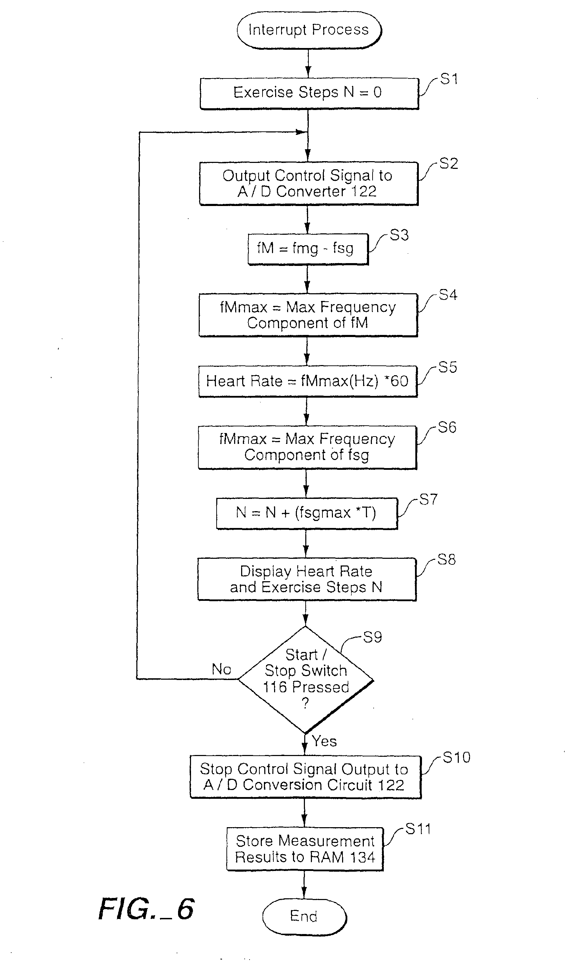

Counting the exercise steps using the acceleration spectrum signal fsg and heart rate measurements using the pulse wave spectrum signal fmg are initiated by pressing the start/stop button switch 116. More specifically, when the CPU 130 detects that the start/stop button switch 116 was pressed it runs the interrupt process shown in Fig. 6.

As shown in the figure the CPU 130 first initializes the number of exercise steps N to 0 (step S1). To get a signal from the frequency analyzer circuit 124, the CPU 130 then outputs a control signal to A/D conversion circuit 122, which is in an inactive state and is activated by the control signal (step S2). The pulse wave signal and the acceleration signal converted to digital signals by the A/D conversion circuit 122 are thus output to the frequency analyzer circuit 124. The frequency analyzer circuit 124 captures the digitized pulse wave signal and acceleration signal for a specific period, applies a FFT process, and outputs the resulting pulse wave spectrum signal fmg and acceleration spectrum signal fsg to the CPU 130.

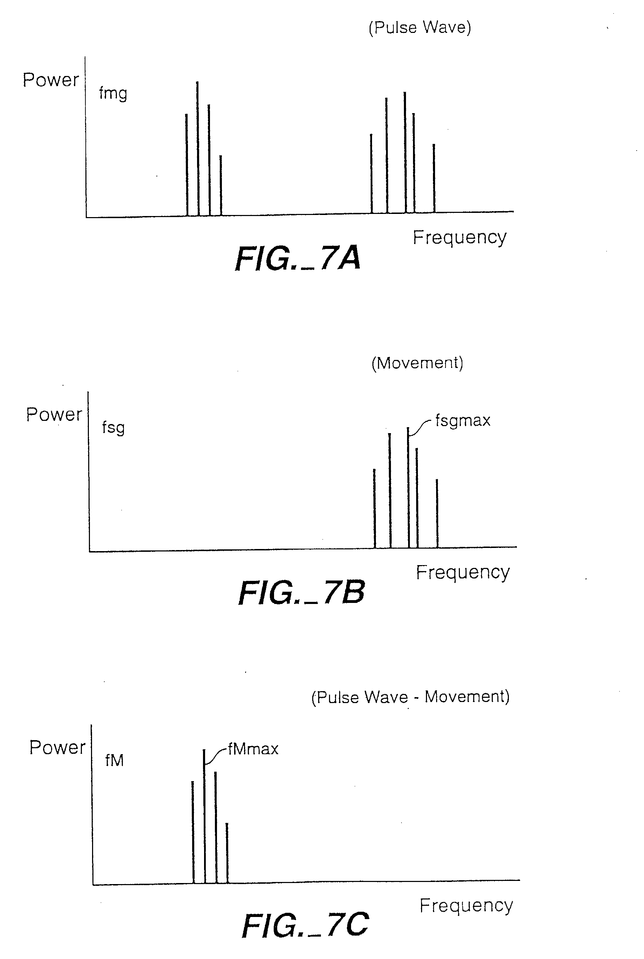

When the CPU 130 receives the pulse wave spectrum signal fmg and acceleration spectrum signal fsg it extracts the pulse wave component to calculate the heart rate. That is, the CPU 130 subtracts the acceleration spectrum signal fsg from the pulse wave spectrum signal fmg to obtain spectrum difference signal fM (step S3). The acceleration spectrum signal fsg is subtracted from the pulse wave spectrum signal fmg for the following reasons. That is, as shown in Fig. 7 (a), the pulse wave spectrum signal fmg detected while exercising contains the acceleration spectrum signal fsg (see Fig. 7 (b) as a frequency component corresponding to body movement (that is, arm movement), and acceleration spectrum signal fsg is subtracted from pulse wave spectrum signal fmg to remove this acceleration spectrum signal fsg component.

The CPU 130 then obtains peak frequency fMmax of which power is highest among the spectrum difference signals fM, as the frequency component equivalent to the pulse wave (step S4). After thus obtaining the pulse wave component (fMmax), CPU 130 substitutes the peak frequency fMmax (that is, pulse wave) obtained in step S3 to equation 1 to calculate the heart rate (beats/minute) (step S5).

$$\text{heart rate (beats/minute)} = \text{peak frequency fMmax (Hz)} * 60 \qquad (1)$$

The CPU 130 then runs the following process to obtain the number of steps from acceleration spectrum signal fsg.

The CPU 130 first obtains the peak frequency fsgmax, of which power is highest among the acceleration apectrum sigmnals fsg as the frequency equivalent to the number of steps per second (step S6). To determine the number of steps walked while this interrupt process runs once (from start to end), the CPU 130 multiplies the time T (sec) corresponding to the run time of the interrupt process by peak frequency fsgmax, and adds the result to the exercise steps N, that is, the cumulative number of steps up to the previous operation (step S7). The CPU 130 then displays the heart rate calculated in step S5 and the exercise steps N calculated in step S7 on LCD 108 (step S8).

The CPU 130 then detects whether the start/stop button switch 116 was pressed to stop heart rate measurement (step S9). If it was not pressed, CPU 130 loops back to step S2 to continue measuring the heart rate and counting the number of steps. If the start/stop button switch 116 was pressed (step S9 returns yes), CPU 130 stops outputting a control signal to A/D conversion circuit 122 (step S10), and thus stops the operation of A/D conversion circuit 122. The CPU 130 then stores the counted exercise steps and heart rate measurement correlated to the date and time of the measurement in RAM 134 (step 511), and the process ends.

The measurement results are also stored with the time that the start/stop button switch 116 was pressed to start measurement (the exercise start time) and how long the interrupt process ran (the measurement time), and these are further described below. It should be noted that while the number of exercise steps N is obtained in step S7 after determining the heart rate in step S5 in the process described above, the heart rate could be determined after detecting the exercise steps N.

The actual operation of the physical activity measurement apparatus 1 while it is in use is described in detail next.

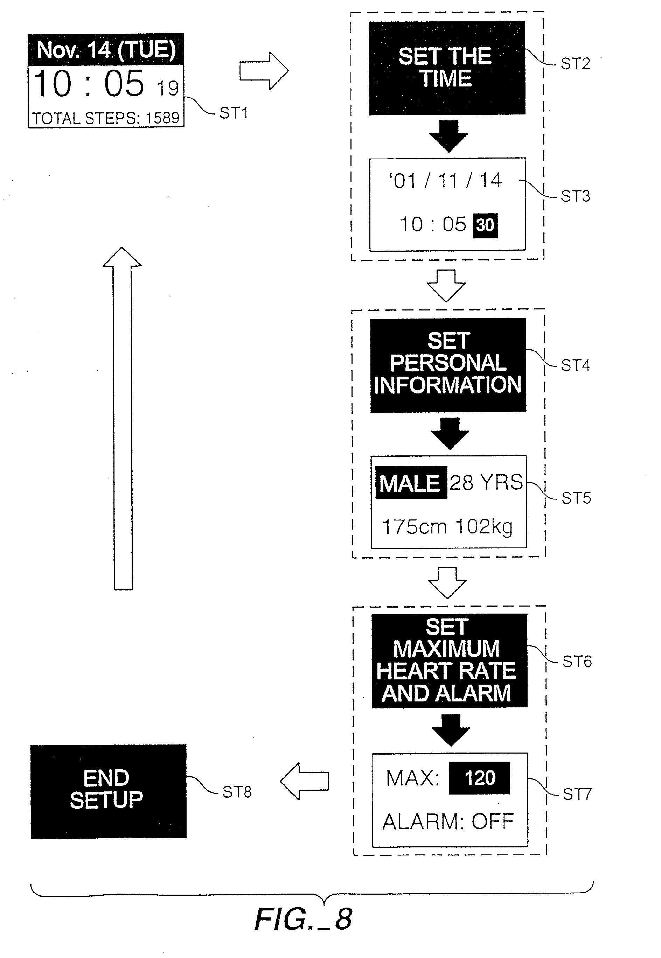

The user must first input the date, current time, and personal information, and set the alarm, the first time the physical activity measurement apparatus 1 is used. More specifically, when the standard display ST1 in which the date, time, and total steps in the day are shown is displayed on the LCD 108 as shown in Fig. 8, and the CPU 130 detects that the user has pressed button switch 111 a specified time (such as 3 seconds), the CPU 130 presents a time input prompt display ST2 on the LCD 108. The time input prompt display ST2 prompts the user to set the current time, and is presented with an inverted high contrast display to make it easier for the user to determine what operations to perform next. Note that each of the other prompts and messages displays described below is likewise presented with an inverted display.

After presenting the time input prompt display ST2, CPU 130 presents the time input display ST3. The value to be input is highlighted by inverting the display as indicated by the seconds place in the time input display ST3 in Fig. 8. The year part of the date is highlighted the first time the time input display ST3 is displayed, and the user first sets the date and then the time. Button switches 112 and 113 are pressed to change the highlighted value. More specifically, the CPU 130 increments the selected value each time button switch 112 is pressed, and decrements the value each time button switch 113 is pressed. By Pressing the selector switch 111, the CPU 130 highlights the value to be set next. The user repeats these operations to sequentially set the date and the time until the seconds are also set.

When the button switch 111 is pressed with the last value to be set in the time input display ST3 highlighted (the seconds in this example), the CPU 130 stores the adjusted current date and time to RAM 134.

To prompt the user to input the personal information, the CPU 130 then presents the personal information input prompt display ST4 on LCD 108, and then presents the personal information input display ST5.

The personal information requested in the personal information input display ST5 is the user's sex, age, height, and weight in this embodiment. Each value is set using the same sequence of operations used in the time input display ST3.

When the button switch 111 is pressed with the last value to be set in the personal information input display ST5 highlighted (the weight in this example), the CPU 130 stores the personal information (sex, age, height, and weight) input by the user to RAM 134.

To prompt the user to input the maximum heart rate and set the alarm, the CPU 130 then presents the maximum heart rate and alarm setting input prompt display ST6 on the LCD 108 and then presents the maximum heart rate and alarm setting display ST7.

The maximum heart rate at which an alarm should be emitted for the user, and whether to sound the alarm when the maximum heart rate is reached, are set in the maximum heart rate and alarm setting display ST7. The maximum heart rate is set to the heart rate prescribed by the user's physician based on the condition of the user. The prescribed heart rate is indicated with both a maximum heart rate and minimum heart rate, and exercise with the heart rate between the maximum heart rate and minimum heart rate results in appropriate exercise that does not put an excessive burden on the heart.

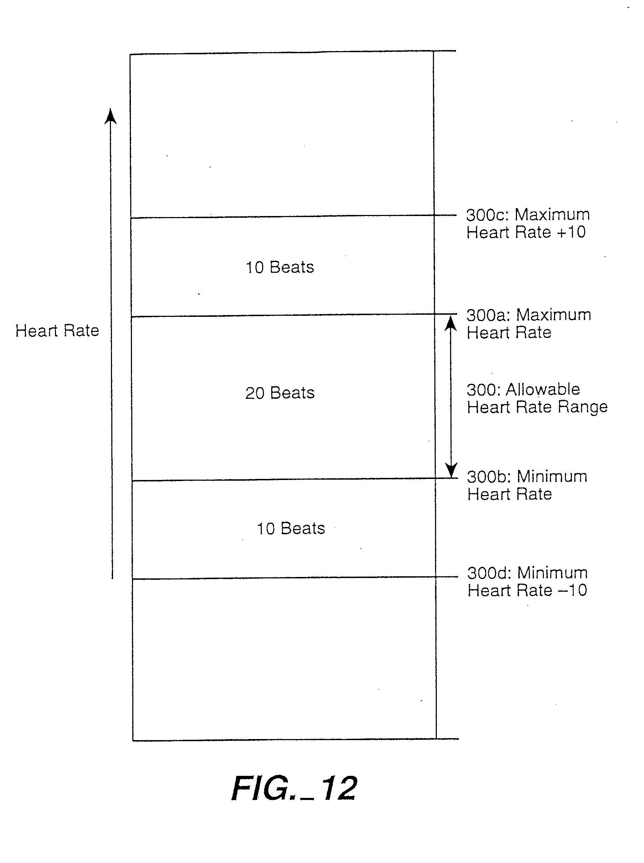

Fig. 12 shows an example of the prescribed heart rate. As shown in the figure the difference between the maximum heart rate (below referred to as maximum heart rate 300a) and the minimum heart rate (minimum heart rate 300b below) is 20 beats. This difference between the maximum heart rate 300a and minimum heart rate 300b is referred to below as the allowable heart rate range 300. The user therefore sets the maximum heart rate 300a as the highest allowable heart rate in the maximum heart rate and alarm setting display ST7.

After setting the maximum heart rate 300a the user selects whether the alarm should sound when the user's heart rate exceeds the set maximum heart rate 300a by pressing button switch 112. By enabling the alarm to be selectively turned on or off, the alarm can be turned off so as to not startle users, such as the elderly, with a weak heart.

When the button switch 111 is pressed after turning the alarm on or off the CPU 130 stores the maximum heart rate 300a and the alarm on/off set by the user to RAM 134. The CPU 130 also stores the minimum heart rate 300b, determined by subtracting 20 beats (equivalent to allowable heart rate range 300) from maximum heart rate 300a, to RAM 134.

The CPU 130 then displays the standard display ST1 again on LCD 108 and thus completes the data input and setup process.

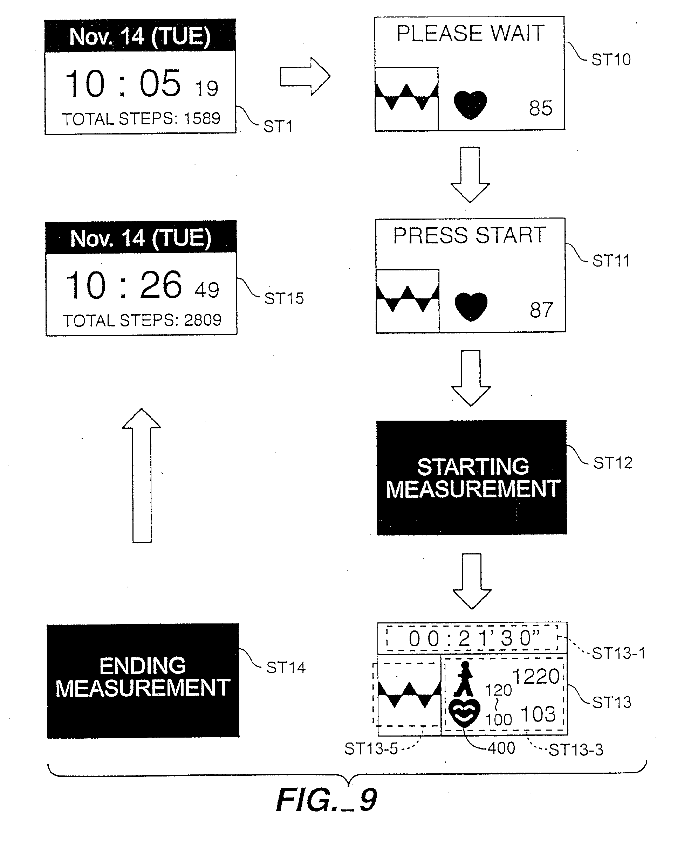

To measure the heart rate while exercising, the user presses the start/stop button switch 116 when the standard display ST1 is displayed on LCD 108. When the CPU 130 detects that start/stop button switch 116 was pressed, it starts the interrupt process described above (see Fig. 6), and thus starts measuring the heart rate and counting the exercise steps. As shown in Fig. 9, the CPU 130 displays a heart rate measurement setup display ST10 on LCD 108 until the fluctuation in the heart rate output from frequency analyzer circuit 124 settles to within a specified range.

After the heart rate fluctuation settles to within this specified range, CPU 130 displays a start measurement prompt display ST11 on LCD 108 prompting the user to press the start/stop button switch 116 to start operation. When the CPU 130 then detects that the user pressed the start/stop button switch 116, it displays a measurement start display ST12 notifying the user that operation started, then runs the above-described interrupt process, and displays measurement display ST13 showing the measured heart rate and exercise steps.

More specifically, the measurement display ST13 has an elapsed time display area ST13-1 showing the time elapsed since the user started exercising, a measurement display area ST13-3 showing the measured heart rate and exercise steps, and a pulse wave display area ST13-5 showing the pulse wave. The exercise steps are displayed to the right beside an icon showing a person walking on the top row of the measurement display area ST13-3, and shows a heart icon 400 and the heart rate on the bottom row. The heart icon 400 changes according to the heart rate.

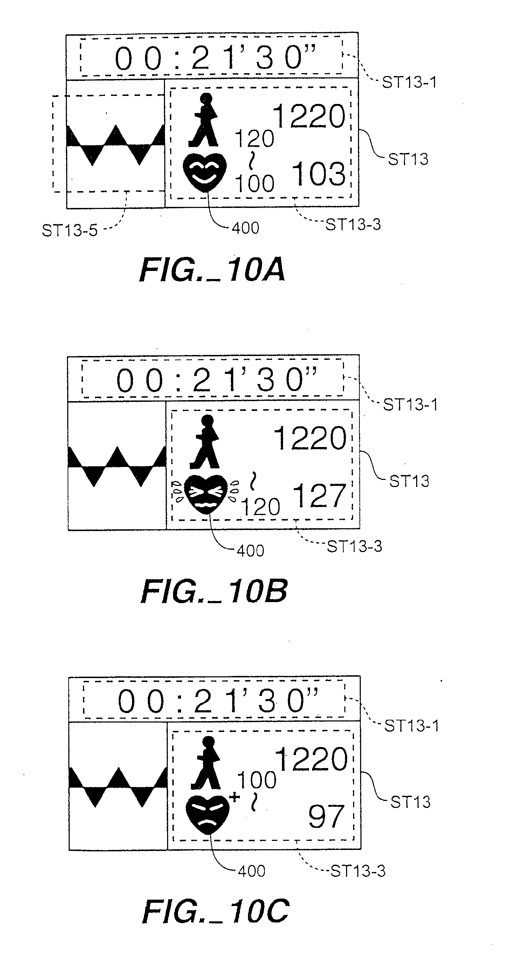

More specifically, a heart icon 400 with a smile is displayed as shown in Fig. 10 (a) when the heart rate is within the allowable heart rate range 300. When the user's heart rate exceeds the maximum heart rate 300a, a heart icon 400 with a distressed expression is displayed as shown in Fig. 10 (b), and when the user's heart rate drops below the minimum heart rate 300b a heart icon 400 with an angry expression is displayed as shown in Fig. 10 (c). By thus varying the heart icon 400 according to whether the user's heart rate is within, above, or below the allowable heart rate range 300, the user can determine the load on the heart by simply glancing at the heart icon 400 in LCD 108. This enables the user to adjust the intensity of the exercise according to the expression of the heart icon 400, and can thereby reliably exercise at the level prescribed by the physician.

Recent studies suggest that training (exercising) at a high intensity level is an effective way of improving heart function. By indicating in the display when the heart rate of the user (particularly patients with heart disease) has dropped below the minimum heart rate 300b, the user can know when he is not exercising enough and can adjust the intensity of the physical activity appropriately.

The user presses the start/stop switch 116 to stop measurement when finished exercising. When the CPU 130 detects that the start/stop button switch 116 was pressed again, it stops outputting control signals to the A/D conversion circuit 122 to stop outputting pulse wave signals and acceleration signals from the A/D conversion circuit 122 to the frequency analyzer circuit 124. The CPU 130 then stores the measurement results to RAM 134, displays a measurement finish display ST14 telling the user that measurement ended, and then displays the standard display ST15. Note that the total steps displayed in the standard display ST15 is now the sum of the total steps before exercising plus the exercise steps.

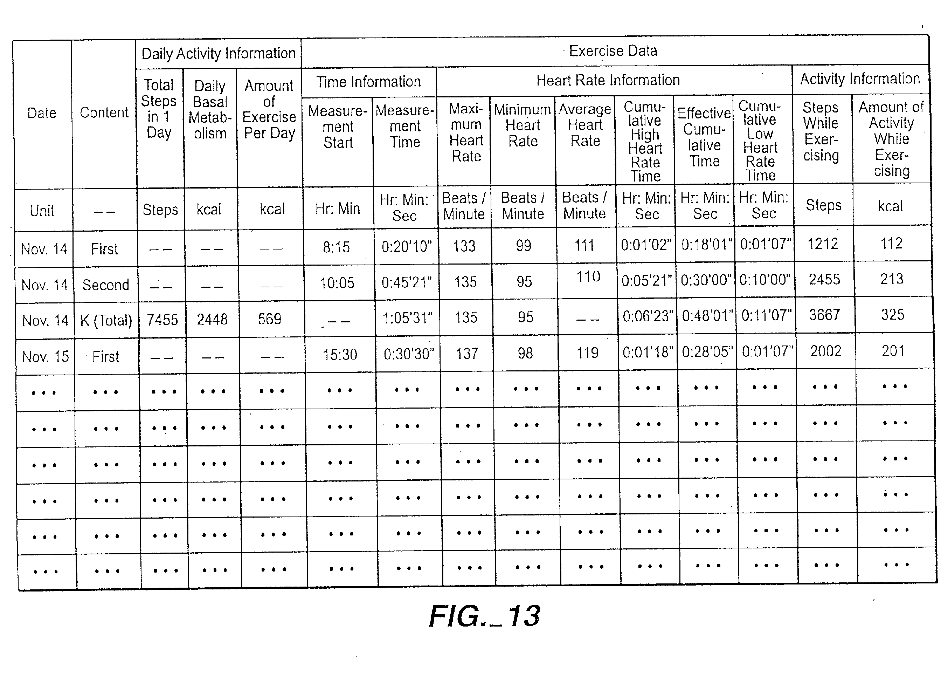

As shown in Fig. 13 the date and content indicator are stored with the results of each measurement. The content indicator denotes whether particular results were recorded for the first, second, or n-th time the user exercised that day, or whether the entry records the exercise totals (denoted with a "k" in this example) for each day. The daily activity information and exercise information are also correlated to the date in the measurement results.

The exercise information denotes the results obtained each time the user exercises, and includes time, heart rate, and activity information. The time information includes the time when measurement (exercise) started and how long measurement (exercise) continued.

The heart rate information includes the maximum heart rate, minimum heart rate, and average heart rate measured. The heart rate information also includes the effective cumulative time the user's heart rate was within the allowable heart rate range 300 while exercising, the cumulative high heart rate time, that is, how long the user's heart rate was within 10 beats above the maximum heart rate 300a, and the cumulative low heart rate time, that is, how long the user's heart rate was within 10 beats below the minimum heart rate 300b.

The activity information includes the number of steps taken while exercising, and the amount of activity while exercising. How the amount of activity is determined is further described below.

The time measurement started is included in the results so that the physician can correlate the results to drugs taken by the user and carbohydrate metabolism.

The effective cumulative time, cumulative high heart rate time, and cumulative low heart rate time are included to evaluate how long the user exercised at what level of intensity.

If the recorded content is the total for one day, the activity information for the day includes the total steps for one day, basal metabolism for one day, and the amount of exercise for one day as shown in Fig. 13. The total steps for one day is the sum of the number of steps while exercising that day and the number of steps taken while not exercising. The amount of exercise for one day is the total amount of exercise while exercising that day and the amount of exercise while not exercising.

If the content field for the entry indicates the total of each exercise, the following information is recorded for each item in the exercise information and activity information. The time information in this case records the total time measurements were taken while exercising that day. The maximum heart rate of the heart rate information records the highest heart rate and the minimum heart rate records the lowest heart rate each time the user exercised. The corresponding totals for each time the user exercised are likewise recorded to the effective cumulative time, cumulative high heart rate time, and cumulative low heart rate time fields. The total number of steps while exercising that day is recorded as the exercise steps of the activity information, and the total amount of exercise while exercising that day is recorded as the amount of exercise while exercising.

It should be noted that RAM 134 preferably has sufficient capacity to store these results from a specified number of days. This specified number of days is preferably equivalent to the number of days (such as one week) between the user's (patient's) visits to the doctor. This enables the physician to monitor how much exercise the user (patient) is getting. The invention shall obviously not be so limited, however, and RAM 134 can be configured to store several weeks of results organized by week, thus making it easier for the user to manage the results in week units.

The amount of exercise is obtained using the following equation.

$$\text{exercise (kcal)} \approx \text{METS * weight (kg) * exercise time (hour)} \qquad (2)$$

where METS (metabolic equivalents) is a coefficient denoting exercise as a multiple of energy consumption when at rest. METS is a common unit established by the American College of Sports Medicine (ACSM) for measuring the intensity of exercise. More specifically, METS is the ratio to oxygen uptake during exercise based on oxygen uptake at rest of 3.5 ml/kg/min. The relationship between METS and energy consumption is shown by the following equation.

$$1 \text{ METS} \approx 1 \text{ kcal/kg/hour} \qquad (3)$$

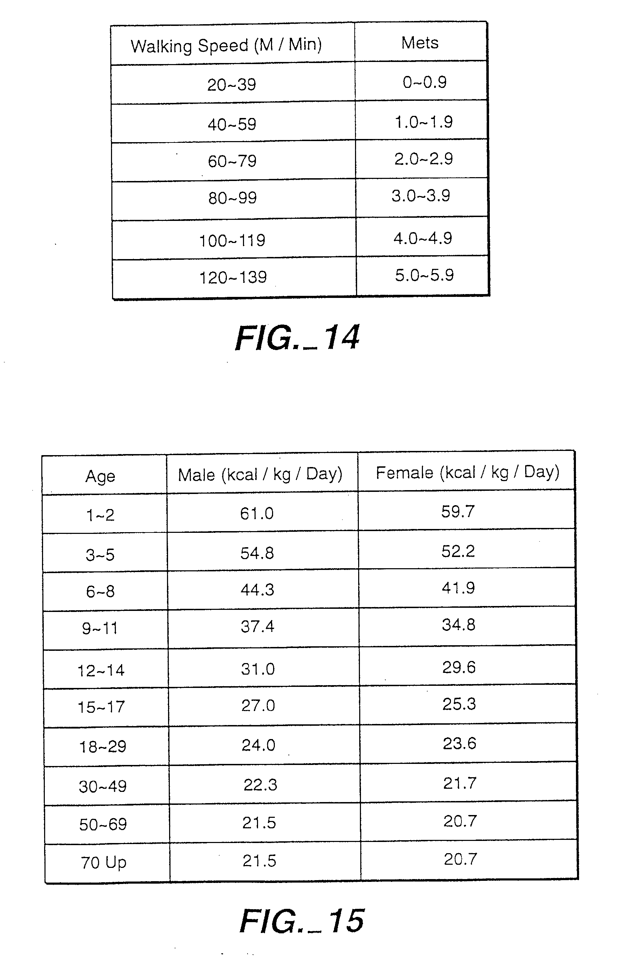

METS is obtained from walking speed, and values calculated to enable easy conversion between walking speed and METS as published by the ACSM are shown in Fig. 14. It is therefore possible to determine METS when the user exercises and thus determine the amount of exercise by calculating the walking speed while exercising. Walking speed is determined from equation (4).

$$\text{walking speed} = \text{length of pace * number of steps / walking}$$
$$\text{time} \qquad (4)$$

$$\text{length of pace} \approx \text{height (cm) - 100 (cm)} \qquad (5)$$

The walking speed from equation (4) is the distance walked per walking (exercise) time unit, and is calculated from the length of one pace and the number of steps. Note that the length of one pace is the distance from heel to heel in one step. Equation (4) is commonly used. The length of one pace from equation (5) is a simple estimation that is generally applicable to adults when walking normally. Energy consumption while exercising can be calculated more precisely by inputting the user's actual pace.

The user's pace is thus calculated in equation (5) from the user-defined height, and the walking speed is calculated from equation (4) using this pace, the measured exercise steps, and how long the user exercises. The METS of the exercise is thus determined from the correlation between walking speed and METS shown in Fig. 14, and the amount of exercise is obtained from equation (2). The CPU 130 calculates the amount of exercise as described above, and stores the measurement results, including the amount of exercise, to RAM 134.

It should be noted that the physical activity measurement apparatus 1 according to this embodiment of the invention calculates the total steps and total energy consumption when the current time is 12:00 midnight. More specifically, when the CPU 130 detects that the current time is 12:00 midnight, it stores the total steps counted to that time together with the date in RAM 134, resets the number of steps displayed in standard display ST1, and starts counting the number of steps from 0 again. The CPU 130 also calculates total energy consumption and stores it with the date in RAM 134. Total energy consumption is determined as shown in the following equation (6) from the basal metabolism and amount of exercise.

$$\text{total energy consumption (kcal)} = \text{basal metabolism + amount}$$
$$\text{of exercise} \qquad (6)$$

The basal metabolism is the minimum energy metabolism physiologically required to maintain the body, and is obtained from equation (7).

$$\text{basal metabolism (kcal)} = \text{standard basal metabolism}$$

$$\text{(kcal/kg/day)} * \text{weight (kg)} \tag{7}$$

The standard basal metabolism is obtained as follows. Fig. 15 shows standard basal metabolism values classified by age and sex as reported in June 1999 by the Japanese Ministry of Health and Welfare (currently the Ministry of Health, Labour, and Welfare) in the Recomended Dietary Allowances for the Japanese - 6th Revision-.

The standard basal metabolism is determined from the age and sex set by the user in the personal information by referencing the table in Fig. 15. The basal metabolism is then determined by substituting this standard basal metabolism and the similarly defined user weight into equation (7). The CPU 130 then applies equation (6) using the resulting basal metabolism and the amount of exercise determined at the end of heart rate measurement to determine the total energy consumption, which it then stores with the date in RAM 134. It should be noted that the calculated daily total energy consumption is preferably stored in RAM 134 in week units similarly to the amount of exercise. It should also be noted that while this embodiment is described calculating total energy consumption from the number of steps as an indicator of movement, total energy consumption can also be calculated from the heart rate as an indicator of physical activity.

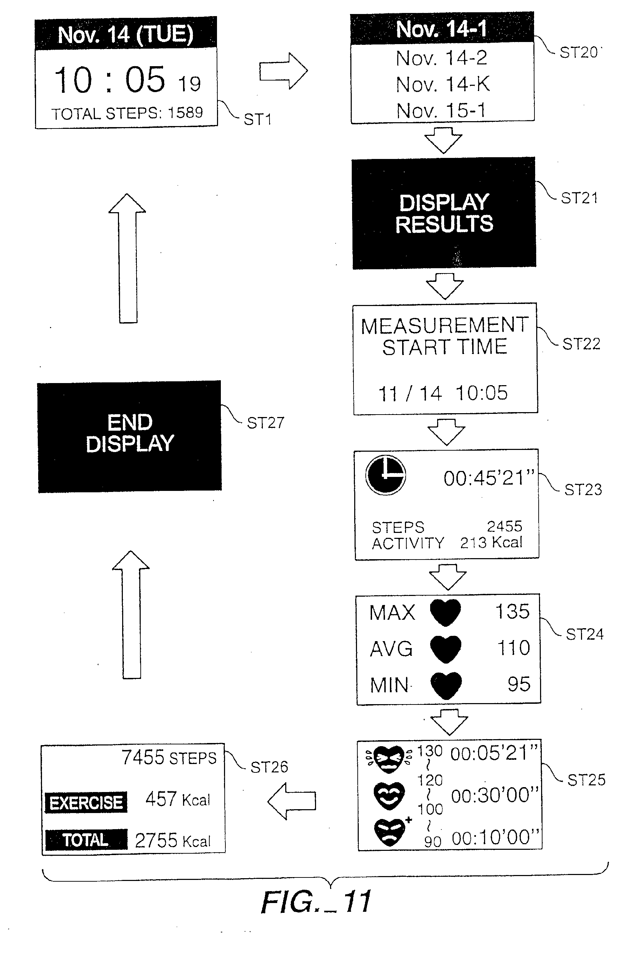

To display the measurement results and other information stored to RAM 134 on LCD 108, the user presses button switch 112 when the standard display ST1 is displayed. As shown in Fig. 11, this causes the CPU 130 to display a date selection display ST20 prompting the user to select a date for which the results are to be displayed. In the example shown in Fig. 20 "Nov 14-1" shown in the date selection screen ST20 denotes the results from the first time the user exercised on November 14, "Nov 14-2" denotes the results from the second time the user exercised on November 14, and "Nov 14-K" denotes the total for all exercise on November 14.

When the user then presses button switch 112 to select the highlighted date, CPU 130 displays a result notification display ST21 on LCD 108 indicating that the measurement results for the selected date will be displayed, and then presents the measurement start time display ST22. The time that exercise started (i.e., that measurement started) is displayed in the measurement start time display ST22.

The CPU 130 successively presents the exercise steps display ST23, heart rate display ST24, cumulative time display ST25, and total energy consumption display ST26 on LCD 108 each time the user presses button switch 112.

The measurement time (how long the user exercised), number of steps while exercising, and amount of exercise (213 kcal in this example) are displayed in the exercise steps display ST23. The highest heart rate, lowest heart rate, and average heart rate are shown in the heart rate display ST24. The cumulative high heart rate time, effective cumulative time, and the cumulative low heart rate time are displayed in the cumulative time display ST25. The total energy consumption display screen ST26 is presented only when the user selects the result totals for the day in date selection display ST20. The total number of steps, amount of exercise, and total energy consumption are displayed for the selected date in the total energy consumption display ST26.

If the user presses button switch 112 when the total energy consumption display screen ST26 is on LCD 108, CPU 130 presents a result notification display ST27 on LCD 108 indicating the result display is ending, and then restores the standard display ST1.

It will thus be apparent that a physical activity measurement apparatus 1 according to this embodiment of the invention measures the heart rate while exercising and the number of steps taken while exercising, and determines the total number of steps per day. It also calculates the amount of exercise for the user from the number of steps while exercising, and the total energy consumption of the user from the total number of steps in a day. It is therefore possible to show to the user the user's heart rate while exercising as an indicator of organic activity, the number of steps as an indicator of physical movement, and total energy consumption per day. The user can therefore determine from the heart rate displayed while exercising how much of a load is being put on the heart (that is, the intensity of the exercise), and can determine the total amount of activity throughout the day from the total number of steps and total energy consumption.

* Alternative embodiment

One embodiment of the present invention is described above by way of example only, and it will be apparent that the invention can be modified in various ways without departing from the scope of the accompanying claims. Some possible alternative embodiments are described next below.

(Variation 1)

The number of steps taken while walking is measured to monitor user movement in the embodiment described above. The number of repetitions of any exercise involving repeating a particular action can be used, however, including chin-ups, situps, and jumping rope.

An acceleration sensor unit 140 is also used above as a means for detecting movement, but the invention shall not be so limited. It is also possible, for example, to calculate the number of steps from the distance moved by the user based on range information detected using a GPS (Global Positioning System) receiver.

Movement shall also not be limited to detecting the number of steps. The physical activity measurement apparatus 1 could have a pressure sensor, for example, for detecting user movement based on the change in pressure such as when the user goes mountain climbing or diving into water.

(Variation 2)

While the user sets the maximum heart rate 300a in the physical activity measurement apparatus 1 described above, it is alternatively possible to set a target for the average heart rate (the "target average heart rate" below). When the target average heart rate is set by the user, the physical activity measurement apparatus 1 calculates and stores the allowable heart rate range 300, maximum heart rate 300a, and minimum heart rate 300b from the set target rate. As described in the above embodiment, the physical activity measurement apparatus 1 changes the expression of the heart icon 400, for example, when measuring the user's heart rate to indicate what range the user's heart rate is in, and calculates the cumulative high heart rate time, effective cumulative time, and the cumulative low heart rate time. When calculating the average heart rate the physical activity measurement apparatus 1 could also calculate the average heart rate when the measured heart rate exceeds the maximum heart rate 300a, the average heart rate when it is within the allowable heart rate range 300, and the average heart rate when below the minimum heart rate 300b.

(Variation 3)

The invention is described above with the heart rate range divided into three subranges as shown in Fig. 12, but the invention shall not be so limited. Two ranges above and below the target average heart rate described in variation 2 above could be used, for example, or the heart rate range could be segmented into more than three ranges. As shown in Fig. 12, a further range to 10 beats above the maximum heart rate 300a, and another range to 10 beats below the minimum heart rate 300b, could also be used. This enables the user to determine even more precisely the load on the user's heart, and thus provides a more accurate indicator of the intensity of the exercise.

(Variation 4)

A heart icon 400 determined by the user's heart rate is displayed on the LCD 108 in the above-described embodiment. If a color LCD 108 is used, however, the color of the heart icon 400 could be changed according to the heart rate, or the format (color and size, for example) of the numbers used to display the heart rate could be changed. This can make the indicator even easier to read and recognize, and thus makes it even easier for the user to determine whether the current heart rate is appropriate.

(Variation 5)

The physical activity measurement apparatus 1 described above starts measuring the user's heart rate when the start/stop button switch 116 is pressed. The CPU 130 could, however, detect when the user starts to exercise based on the step detection signal output from the rectangular wave conversion circuit 144, and start measuring the heart rate (that is, run the interrupt process shown in Fig. 6) accordingly.

(Variation 6)

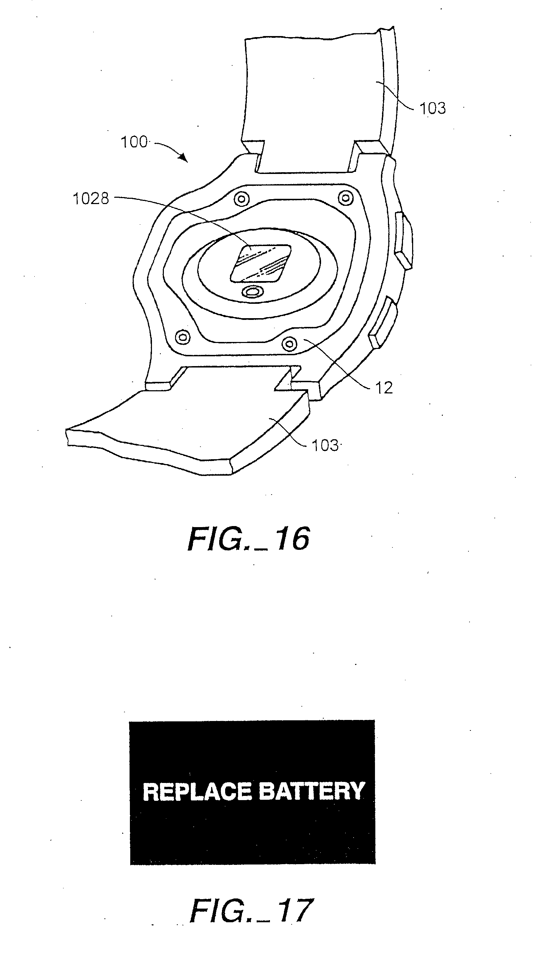

The pulse wave sensor unit 102 of the above-described physical activity measurement apparatus 1 is worn on the user's finger. As shown in Fig. 16, however, the physical activity measurement apparatus 1 could be configured so that pulse waves are detected from the area in contact with the back cover 12 (that is, the back of wrist) when the user wears the physical activity measurement apparatus 1. More specifically in this case a transparent plate 1028 of glass or other material is disposed in substantially the middle of the back cover 12 as shown in Fig. 16, and an LED (not shown in the figure) for emitting light through this transparent plate 1028 is incorporated in the main unit 100. A phototransistor (also not shown in the figure) is also disposed in the main unit 100 to detect reflections through the transparent plate 1028. With this configuration the user only needs to wear the main unit 100.

(Variation 7)

The pulse wave sensor unit 102 is connected to the connector 105 through cable 101 in the above embodiment. The physical activity measurement apparatus 1 could also be connected through connector 105 to a personal computer or other information device. When thus connected measurement results stored to RAM 134 can be transferred from the physical activity measurement apparatus 1 to another device. By connecting the physical activity measurement apparatus 1 to a data processing device used by the physician, the physician can download and read the user's (patient's) measurement results on the data processing device for use developing an appropriate exercise regimen.

(Variation 8)

A battery is incorporated in the physical activity measurement apparatus 1 described above. It is therefore also possible to display a prompt telling the user to replace the battery when battery capacity drops below a certain level as shown in Fig. 17.

(Variation 9)

As also described above the frequency analyzer circuit 124 applies a fast Fourier transform to the pulse wave signal and acceleration signal, derives a pulse wave from the difference between the resulting pulse wave spectrum signal fmg and acceleration spectrum signal fsg, and thereby obtains the heart rate. It is alternatively possible to extract only the pulse wave component and obtain the heart rate by time-frequency analyzing the pulse wave signal using a time-frequency analysis circuit. A Wavelet transform process or Wigner-Ville distribution can be used for the time-frequency analysis process.

It will thus be obvious that a physical activity measurement apparatus according to the present invention can be used to record the user's physical activity and amount of activity.

Although the present invention has been described in connection with the preferred embodiments thereof with reference to the accompanying drawings, it is to be noted that various changes and modifications will be apparent to those skilled in the art. Such changes and modifications are to be understood as included within the scope of the present invention as defined by the appended claims.

**Claims**

**1.** A physical activity measurement apparatus comprising:

a movement detection means for detecting user movement;
a physiological reaction detection means for detecting a physiological reaction of the user;
a physical activity data calculating means for calculating physical activity data for the user from detection results output by the physiological reaction detection means;
an evaluation means for determining if the user started exercising; and
storage means for storing detection results from the movement detection means and physical activity data from when the evaluation result becomes positive.

**2.** A physical activity measurement apparatus as claimed in claim 1, wherein the physical activity data calculating means calculates physical activity data for the user from detection results output by the movement detection means and physiological reaction detection means.

**3.** A physical activity measurement apparatus as claimed in claim 2, wherein the storage means also stores detection results from the movement detection means from when the evaluation result becomes positive.

**4.** A physical activity measurement apparatus as claimed in any of claims 1 to 3, further comprising an input capturing means for capturing input from the user;

the evaluation means determining that the user started exercising when the input capturing means detects input from the user.

**5.** A physical activity measurement apparatus as claimed in any of claims 1 to 3, wherein the evaluation means

**EP 1 369 082 A2**

determines if the user started exercising based on a detection result from the movement detection means.

**6.** A physical activity measurement apparatus as claimed in any of claims 1 to 3, wherein the storage means stores a predefined number of days of daily detection results from the movement detection means and one day of physical activity data from when the evaluation result becomes positive.

**7.** A physical activity measurement apparatus as claimed in claim 6, wherein the storage means further stores one day of detection results from the movement detection means from when the evaluation result becomes positive.

**8.** A physical activity measurement apparatus as claimed in claim 7, wherein the storage means stores daily detection results from the movement detection means, one day of physical activity data from when the evaluation result becomes positive, and one day of detection results from the movement detection means from when the evaluation result becomes positive, correlated to the date.

**9.** A physical activity measurement apparatus as claimed in any of claims 1 to 3, further comprising a notification means for reporting detection results from the movement detection means until the evaluation result becomes positive, and reporting the physical activity data after the evaluation result becomes positive.

**10.** A physical activity measurement apparatus as claimed in claim 9, wherein after the evaluation result becomes positive the notification means further reports detection results from the movement detection means from when the evaluation result becomes positive.

**11.** A physical activity measurement apparatus as claimed in claim 1, wherein the movement detection means comprises an acceleration detection means for detecting acceleration based on the movement, and a number of steps calculating means for determining the number of user steps based on the detected acceleration;
the physiological reaction detection means detects a pulse wave from the user; and
the physical activity data calculating means calculates the heart rate as the physical activity data from the detected pulse wave.

**12.** A physical activity measurement apparatus as claimed in claim 2 or 3, wherein the physical activity data calculating means calculates the heart rate from the detected pulse wave and acceleration.

**13.** A physical activity measurement apparatus as claimed in claim 11 or 12, further comprising:

a heart rate evaluation means for detecting which of multiple heart rate ranges the heart rate calculated by the physical activity data calculating means is in; and
a cumulative time calculation means for determining for each of the multiple heart rate ranges the cumulative time the calculated heart rate is within each range based on the evaluation result from the heart rate evaluation means;
the storage means further storing multiple heart rate ranges and storing the calculated cumulative times for each of the multiple heart rate ranges.

**14.** A physical activity measurement apparatus as claimed in claim 13, wherein the storage means stores the total steps per day calculated by the number of steps calculating means and the cumulative time per day for each of the multiple heart rate ranges accumulated over a predefined number of days.

**15.** A physical activity measurement apparatus as claimed in claim 14, wherein the storage means further stores the number of steps while exercising for one day from when the evaluation result becomes positive.

**16.** A physical activity measurement apparatus as claimed in claim 13, further comprising a display means for displaying a graphic according to the evaluation result of the heart rate evaluation means.

**17.** A physical activity measurement apparatus as claimed in claim 13, further comprising a heart rate range calculation means for determining a plurality of heart rate ranges according to a set heart rate that is set by the user.

**18.** A physical activity measurement apparatus as claimed in claim 17, wherein the set heart rate is a prescribed maximum heart rate; and
the heart rate range calculation means determines the plural heart rate ranges from the maximum heart rate

13

and the pulse count width of the prescribed heart rate.

**19.** A physical activity measurement apparatus as claimed in any of claims 1 to 3, further comprising an amount of exercise calculation means for determining the amount of user exercise from detection results output by the movement detection means and user-defined personal information;

the storage means further storing personal information including physical attributes of the user, and storing a predefined number of days of amount of exercise data calculated per day.

**20.** A physical activity measurement apparatus as claimed in any of claims 1 to 3, further comprising an amount of exercise calculation means for determining the amount of user exercise from physical activity data from when the evaluation result of the evaluation means becomes positive and user-defined personal information;

the storage means further storing personal information including physical attributes of the user, and storing a predefined number of days of amount of exercise data calculated per day.

**21.** A physical activity measurement apparatus as claimed in any of claims 19 or 20, further comprising a basal metabolism determination means for determining the user's basal metabolism from a correlation table and user-defined personal information; and

a total energy consumption calculation means for calculating total energy consumption per day from the amount of exercise calculated by the amount of exercise calculation means and the basal metabolism determined by the basal metabolism determination means;

the storage means further storing a correlation table correlating basal metabolism, sex, and age information, and storing a predefined number of days of calculated total energy consumption data.

**22.** A physical activity measurement apparatus as claimed in any of claims 6, 7, 15, 19, 20, or 21, wherein the predefined number of days is a unit of seven days.

*FIG.\_1A*

*FIG.\_1B*

FIG._2

FIG._3

FIG._4

FIG._5

( Interrupt Process )

Exercise Steps N = 0 — S1

Output Control Signal to
A / D Converter 122 — S2

$fM = fmg - fsg$ — S3

fMmax = Max Frequency
Component of fM — S4

Heart Rate = fMmax(Hz) *60 — S5

fMmax = Max Frequency
Component of fsg — S6

$N = N + (fsgmax *T)$ — S7

Display Heart Rate
and Exercise Steps N — S8

Start /
Stop Switch
116 Pressed
? — S9

No

Yes

Stop Control Signal Output to
A / D Conversion Circuit 122 — S10

Store Measurement
Results to RAM 134 — S11

( End )

*FIG._6*

(Pulse Wave)

Power | fmg

Frequency

**FIG._7A**

(Movement)

Power | fsg

fsgmax

Frequency

**FIG._7B**

(Pulse Wave - Movement)

Power | fM

fMmax

Frequency

**FIG._7C**

**Nov. 14 (TUE)**
**10 : 05** 19
TOTAL STEPS: 1589 — ST1

⇒

**SET THE TIME** — ST2

⬇

'01 / 11 / 14
10 : 05 **30** — ST3

⇓

**SET PERSONAL INFORMATION** — ST4

⬇

**MALE** 28 YRS
175cm 102kg — ST5

⇓

**SET MAXIMUM HEART RATE AND ALARM** — ST6

⬇

MAX: **120**
ALARM: OFF — ST7

⇐

**END SETUP** — ST8

*FIG._8*

Nov. 14 (TUE)
10 : 05 19
TOTAL STEPS: 1589 — ST1

PLEASE WAIT — ST10
85

Nov. 14 (TUE)
10 : 26 49 — ST15
TOTAL STEPS: 2809

PRESS START — ST11
87

STARTING MEASUREMENT — ST12

ENDING MEASUREMENT — ST14

0 0 : 2 1 ' 3 0 " — ST13-1
1220 — ST13
120
~
100
103
ST13-5
400 — ST13-3

## FIG._9

FIG._10A

FIG._10B

FIG._10C

Nov. 14 (TUE)

# 10 : 05 19

TOTAL STEPS: 1589

ST1

Nov. 14-1
Nov. 14-2
Nov. 14-K
Nov. 15-1

ST20

DISPLAY
RESULTS

ST21

MEASUREMENT
START TIME

11 / 14  10:05

ST22

🕐        00:45'21"

ST23

STEPS         2455
ACTIVITY   213 Kcal

MAX   ❤   135

AVG   ❤   110

MIN   ❤   95

ST24

END
DISPLAY

ST27

7455 STEPS

EXERCISE   457 Kcal

TOTAL   2755 Kcal

ST26

130  00:05'21"
120
100
90   00:30'00"

00:10'00"

ST25

## FIG._11

Heart Rate

10 Beats

20 Beats

10 Beats

300c: Maximum
Heart Rate +10

300a: Maximum
Heart Rate

300: Allowable
Heart Rate Range

300b: Minimum
Heart Rate

300d: Minimum
Heart Rate −10

*FIG._12*

| Date | Content | Daily Activity Information | | | Exercise Data | | | | | | | | | |
|---|---|---|---|---|---|---|---|---|---|---|---|---|---|---|
| | | Total Steps in 1 Day | Daily Basal Metabolism | Amount of Exercise Per Day | Time Information | | Heart Rate Information | | | | | | Activity Information | |
| | | | | | Measurement Start | Measurement Time | Maximum Heart Rate | Minimum Heart Rate | Average Heart Rate | Cumulative High Heart Rate Time | Effective Cumulative Time | Cumulative Low Heart Rate Time | Steps While Exercising | Amount of Activity While Exercising |
| Unit | — — | Steps | kcal | kcal | Hr: Min | Hr: Min: Sec | Beats / Minute | Beats / Minute | Beats / Minute | Hr: Min: Sec | Hr: Min: Sec | Hr: Min: Sec | Steps | kcal |
| Nov. 14 | First | — — | — — | — — | 8:15 | 0:20'10" | 133 | 99 | 111 | 0:01'02" | 0:18'01" | 0:01'07" | 1212 | 112 |
| Nov. 14 | Second | — — | — — | — — | 10:05 | 0:45'21" | 135 | 95 | 110 | 0:05'21" | 0:30'00" | 0:10'00" | 2455 | 213 |
| Nov. 14 | K (Total) | 7455 | 2448 | 569 | — — | 1:05'31" | 135 | 95 | — — | 0:06'23" | 0:48'01" | 0:11'07" | 3667 | 325 |
| Nov. 15 | First | — — | — — | — — | 15:30 | 0:30'30" | 137 | 98 | 119 | 0:01'18" | 0:28'05" | 0:01'07" | 2002 | 201 |
| . . . | . . . | . . . | . . . | . . . | . . . | . . . | . . . | . . . | . . . | . . . | . . . | . . . | . . . | . . . |
| . . . | . . . | . . . | . . . | . . . | . . . | . . . | . . . | . . . | . . . | . . . | . . . | . . . | . . . | . . . |
| . . . | . . . | . . . | . . . | . . . | . . . | . . . | . . . | . . . | . . . | . . . | . . . | . . . | . . . | . . . |
| . . . | . . . | . . . | . . . | . . . | . . . | . . . | . . . | . . . | . . . | . . . | . . . | . . . | . . . | . . . |
| . . . | . . . | . . . | . . . | . . . | . . . | . . . | . . . | . . . | . . . | . . . | . . . | . . . | . . . | . . . |
| . . . | . . . | . . . | . . . | . . . | . . . | . . . | . . . | . . . | . . . | . . . | . . . | . . . | . . . | . . . |

*FIG._13*

EP 1 369 082 A2

| Walking Speed (M / Min) | Mets |
|---|---|
| 20~39 | 0~0.9 |
| 40~59 | 1.0~1.9 |
| 60~79 | 2.0~2.9 |
| 80~99 | 3.0~3.9 |
| 100~119 | 4.0~4.9 |
| 120~139 | 5.0~5.9 |

## FIG._14

| Age | Male (kcal / kg / Day) | Female (kcal / kg / Day) |
|---|---|---|
| 1~2 | 61.0 | 59.7 |
| 3~5 | 54.8 | 52.2 |
| 6~8 | 44.3 | 41.9 |
| 9~11 | 37.4 | 34.8 |
| 12~14 | 31.0 | 29.6 |
| 15~17 | 27.0 | 25.3 |
| 18~29 | 24.0 | 23.6 |
| 30~49 | 22.3 | 21.7 |
| 50~69 | 21.5 | 20.7 |
| 70 Up | 21.5 | 20.7 |

## FIG._15

*FIG._16*

*FIG._17*